# FASCICULE DE BREVET EUROPEEN

(11) **EP 1 502 585 B1**
(45) Date de publication et mention de la délivrance du brevet: **05.06.2013**
(21) Numéro de dépôt: 04291871.4
(22) Date de dépôt: 23.07.2004
(51) Int. Cl.: A61K 8/44, A61K 8/46, A61K 8/73, A61K 8/81, A61Q 5/12

(54) **Compositions cosmétique contenant un mélange de tensioactifs, un mélange de polymères cationiques et une silicone**
Kosmetische Zusammensetzung enthaltend verschiedene Tenside, verschiedene kationische Polymere und ein Silikon
Cosmetic composition containing a surfactant mixture, a cationic polymers mixture and a silicone

(30) Priorité: 28.07.2003 FR 0309240
(43) Date de publication de la demande: 02.02.2005
(73) Titulaire: L'Oréal, 75008 Paris (FR)
(72) Inventeur: Maubru, Mireille, 78400 Chatou (FR); Liebard, Bruno, 92320 Chatillon (FR)
(74) Mandataire: Dossmann, Gérard

(56) Documents cités:
- EP-A- 1 132 079
- WO-A-97/26860
- WO-A-98/50007
- WO-A-99/09947
- FR-A- 2 080 759
- GB-A- 2 255 101
- US-A- 5 726 137

## Description

La présente invention concerne des compositions pour le traitement cosmétique des fibres kératiniques et plus particulièrement des cheveux, contenant un mélange de tensioactifs, un mélange de polymères cationiques particuliers et au moins une silicone particulière, un procédé de traitement cosmétique des fibres kératiniques utilisant de telles compositions.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et/ou les teintures. Il en résulte que les cheveux sont souvent difficiles à démêler ou à coiffer et les chevelures, même abondantes, conservent difficilement une coiffure de bon aspect en raison du fait que les cheveux manquent de vigueur et de nervosité.

On sait protéger les cheveux des effets de la lumière en y appliquant des filtres UV hydrosolubles ou non, polymèriques ou non, des nanoparticules, des antioxydants, des agents complexants de métaux ou chélatants, ou des agents anti-radicaux libres.

Les cheveux fragilisés peuvent être également renforcés mécaniquement en y appliquant certains polymères cationiques seuls ou en mélange avec des électrolytes spécifiques.

Pour mieux démêler et empêcher une certaine rêcheur des cheveux, on peut aussi leur appliquer des agents de conditionnement monomèriques ou polymèriques, hydrosolubles ou non.

Il est aujourd'hui connu d'ajouter ces divers agents de conditionnement en mélange à des compositions de coloration capillaire, à des shampooings ou à d'autres compositions cosmétiques, afin de conférer aux cheveux des propriétés cosmétiques telles que le démêlage, le toucher, le lissage, la brillance et le volume.

Par exemple, la demande de brevet WO 97/26860 concerne une composition de shampooing conditionnant qui permet un démêlage des cheveux mouillés ou secs plus facile, et surtout qui n'irrite ni les yeux ni les peaux sensibles. Cette composition comprend un mélange de tensioactifs et un mélange d'agents de conditionnement.

La demande de brevet GB-A-2255101 décrit des shampoings comprenant un mélange de tensioactifs anioniques, amphotères et non ioniques, un dérivé siliconé non aminé et des agents conditionnant cationiques.

Le document US 5726137 décrit de manière générale une composition de shampoing conditionnant à faible teneur en silicone contenant un mélange des tensioactifs anioniques, amphotères et au moins un polymère cationique.

Toutefois, les compositions contenant des agents de conditionnement en mélange présentent des problèmes de stabilité. Il existe un réel besoin de compositions cosmétiques présentant une grande stabilité et des effets de conditionnement améliorés.

La demande de brevet WO 94/06403 propose de résoudre ce problème de stabilité. Cette demande porte sur une composition de shampooing conditionnant pour les cheveux dans laquelle les agents ayant des propriétés de conditionnement et les agents habituellement utilisés dans les shampooings sont stables. Cette composition stable contient un tensioactif anionique, un polymère vinylique cationique, un agent de conditionnement pour les cheveux, un agent dispersant permettant de stabiliser la composition et de l'eau.

La demande de brevet japonaise 46-312915 porte sur une composition pour le traitement des cheveux à base de gomme de guar quaternisée et de polymère cationique. Ce type de composition permet d'obtenir des effets de conditionnement uniformes sur les cheveux comme par exemple des cheveux mouillés plus lisses lors du lavage et du rinçage, un aspect non-gras ainsi qu'une plus grande stabilité de la composition dans le temps.

Toutefois, ces compositions ne sont pas suffisamment stables et présentent des inconvénients aussi bien au niveau des qualités d'usage avec une production insuffisante de mousse, que des qualités cosmétiques en entraînant un toucher rêche des cheveux.

La demanderesse a découvert que la combinaison d'un mélange de tensioactifs, d'un mélange de polymères cationiques particuliers et d'une silicone particulière permettait d'obtenir des compositions cosmétiques plus stables que les compositions précédemment décrites, et ayant des qualités d'usage et cosmétiques meilleures. En effet, les compositions selon l'invention permettent d'obtenir une mousse abondante à l'application et une grande douceur des cheveux mouillés. De plus, ces nouvelles compositions apportent aux cheveux bouclés une belle définition de boucles et du lissage.

Un autre objet de l'invention porte sur un procédé de traitement cosmétique des matières kératiniques utilisant les compositions précédemment décrites.

D'autres objets, caractéristiques, aspects et avantages de l'invention apparaîtront encore plus clairement à la lecture de la description et des divers exemples qui suivent.

La présente invention concerne une composition cosmétique pour le traitement cosmétique des fibres kératiniques, telles que les cheveux, comprenant :
- au moins un tensioactif anionique ;
- au moins un tensioactif amphotère ou zwitterionique;
- au moins un premier polymère cationique choisi parmi les polysaccharides cationiques ;
- au moins un second polymère cationique choisi parmi les homo ou copolymères de dialkyldiallyl ammonium ; et
- au moins une silicone non volatile non aminée,
la quantité totale de tensioactifs étant comprise dans la composition entre 4,5 et 20 % du poids total de la composition.

Les tensioactifs anioniques utilisables dans le cadre de la présente invention, sont choisis parmi les sels, en particulier les sels de métaux alcalins, notamment de sodium, les sels d'ammonium, les sels d'amines, les sels d'aminoalcools ou les sels de magnésium ; les composés suivants : les alkylsulfates, les alkyléthersulfates, les alkylamidoéthersulfates, les alkylarylpolyéthersulfates, les sulfates de monoglycérides ; les alkylsulfonates, les alkylphosphates, les alkylamidesulfonates, les alkylarylsulfonates, les α-oléfine-sulfonates, les paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les citrates de alkylglucoside, les tartrates d'alkylpolyglycoside et les sulfosuccinates d'alkylpolyglycoside, les alkylsulfosuccinamates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D-galactoside uroniques et leurs sels, les acides alkyl(C₆-C₂₄)éther carboxyliques polyoxyalkylénés, les acides alkyl(C₆-C₂₄)aryléther carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'alkylène en particulier d'éthylène, et leurs mélanges.

De préférence, les alkylsulfates et les alkyléthersulfates sont utilisés.

Les quantités de tensioactifs anioniques présents dans la composition selon l'invention peut varier de 0,01 à 19% du poids total de la composition.

Les agents tensioactifs amphotères ou zwitterioniques peuvent être notamment des amines ou des ammoniums quaternaires portant une chaîne linéaire ou ramifiée comportant 8 à 18 atomes de carbone et contenant au moins un groupe anionique hydrosolubilisant, comme par exemple un groupe carboxylate, sulfonate, sulfate, phosphate ou phosphonate.

On peut citer encore les alkyl(C₈-C₂₀)bétaïnes, les sulfobétaïnes, les alkyl(C₈-C₂₀)amidoalkyl(C₁-C₆)betaïnes ou les alkyl(C₈-C₂₀)amidoalkyl(C₁-C₆)sulfobétaïnes. Parmi ces produits, on peut notamment citer, plus particulièrement la cocoylbétaïne et la cocoamidopropylbétaïne.

Parmi les amines amphotères, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₁-CONHCH₂CH₂-N⁺(R₂)(R₃)(CH₂COO⁻) (I)

dans laquelle :
R₁ désigne un radical alkyle linéaire ou ramifié en C₅-C₂₀ provenant d'un acide R₁-COOH présent dans l'huile de coprah hydrolysée, un radical heptyle, nonyle ou undécyle, R₂ désigne un groupement β -hydroxyéthyle et R₃ un groupement carboxyméthyle ; et

   R₄-CONH CH₂CH₂-N(B)(C) (II)

   dans laquelle :
   B représente -CH₂CH₂OX, C représente -(CH₂)_{z}-Y, avec z = 1 ou 2,
   X désigne le groupement - CH₂CH₂-COOH ou un atome d'hydrogène,
   Y désigne -COOH ou le radical -CH₂-CHOH-SO₃H,
R₄ désigne un radical alkyle linéaire ou ramifié, saturé ou non en C₅-C₂₀ provenant d'un acide R₄-COOH présent par exemple dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple, on peut citer le cocoamphodiacetate commercialisé sous la dénomination commerciale MIRANOL® C2M concentré par la société RHODIA CHIMIE.

De préférence, les tensioactifs amphotères ou zwitterioniques sont choisis parmi les alkylbétaïnes, les amidoalkylbétaïnes et les alkylamphodiacétate.

Les quantités de tensioactifs amphotères ou zwitterioniques présent dans la composition selon l'invention peuvent varier de 1 à 10% du poids total de la composition.

La quantité totale de tensioactifs présents dans la composition selon l'invention est comprise entre 4,5 à 20% du poids total de la composition.

De préférence, le rapport pondéral entre les tensioactif(s) anionique(s) et le(s) tensioactif(s) amphotère(s) (tensioactif(s) anionique(s)/tensioactif(s) amphotère(s)) est supérieur à 1,5, mieux encore il se situe dans l'intervalle allant de 2 à 3, et encore plus préférentiellement de 2 à 10.

Au sens de la présente invention, l'expression "polymère cationique" désigne tout polymère contenant des groupements cationiques et/ou des groupements ionisables en groupement cationique.

Les polymères cationiques utilisés ont généralement une masse moléculaire moyenne en nombre comprise entre 500 et 5.10⁶ environ, et de préférence comprise entre 10³ et 3.10⁶ environ.

La composition selon l'invention contient au moins un premier polymère cationique choisi parmi les polysaccharides cationiques, en particulier les celluloses cationiques, les gommes de guar cationiques.

Ces polymères cationiques peuvent être des dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.

Ces polymères cationiques peuvent être aussi des dérivés de cellulose cationiques tels que les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, de diméthyl-diallylammonium.

Les produits commercialisés répondant à cette définition sont plus particulièrement les produits vendus sous la dénomination "Celquat L 200" et "Celquat H 100" par la Société National Starch.

Les gommes de guar cationiques sont décrites plus particulièrement dans les brevets US 3 589 578 et 4 031 307 tel que les gommes de guar contenant des groupements cationiques trialkylammonium. On utilise par exemple des gommes de guar modifiées par un sel, par exemple un chlorure, de 2,3-époxypropyl triméthylammonium, de glycidyltriméthylammonium, de 3-chloro-2-hydroxypropyltriméthylammonium.

De tels produits sont commercialisés notamment sous les dénominations commerciales de JAGUAR C13 S, JAGUAR C 15, JAGUAR C 17 ou JAGUAR C162 par la société RHODIA CHIMIE.

De préférence, les polysaccharides cationiques de l'invention sont des dérivés de gomme de guar cationiques.

Dans ces compositions, ces premiers polymères peuvent être présents à une concentration comprise entre 0,01% et 10%, de préférence entre 0,03% et 5%, et encore plus préférentiellement entre 0,05% et 2%, et encore plus préférentiellement entre 0,07% et 1 du poids total de la composition.

La composition selon l'invention contient au moins un second polymère cationique choisi parmi des homo ou copolymères de dialkyldiallyl ammonium.

Les polymères d'alkyldiallylamine ou de dialkyldiallylammonium tels que les homopolymères ou copolymères comportant comme constituant principal de la chaîne des motifs répondant aux formules suivantes (III) ou (IV) : 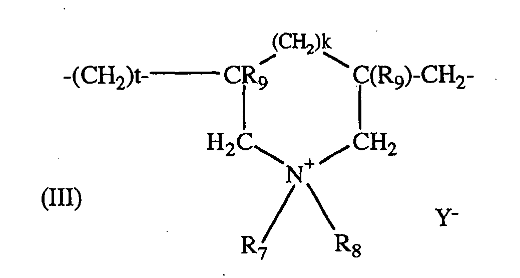 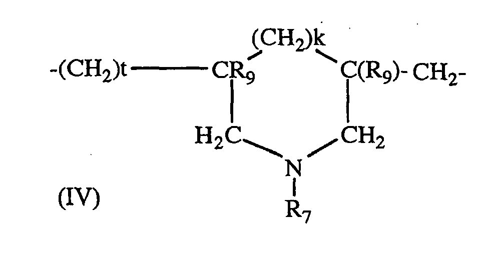 dans lesquelles k et t sont égaux à 0 ou 1, la somme k + t étant égale à 1 ; R9 désigne un atome d'hydrogène ou un radical méthyle ; R7 et R8, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4), ou R7 et R8 peuvent désigner conjointement avec l'atome d'azote auquel ils sont rattachés, des groupement hétérocycliques, tels que pipéridinyle ou morpholinyle ; R7 et R8 indépendamment l'un de l'autre désignent de préférence un groupement alkyle ayant de 1 à 4 atomes de carbone ; Y-est un anion tel que bromure, chlorure, acétate, borate, citrate, tartrate, bisulfite, sulfate, phosphate. Ces polymères sont notamment décrits dans le brevet français 2.080.759 et dans son certificat d'addition 2.190.406.

Parmi les polymères définis ci-dessus, on peut citer plus particulièrement l'homopolymère de chlorure de diméthyldiallylammonium vendu sous la dénomination "Merquat 100" par la société Nalco (et ses homologues de faible masse moléculaire moyenne en poids) et les copolymères de chlorure de diallyldiméthylammonium et d'acrylamide commercialisés sous la dénomination "MERQUAT S" ou "MERQUAT 550".

De préférence, ce second polymère est choisi parmi les homopolymères et les copolymères de chlorure de diallyldiméthylammonium, encore plus particulièrement les copolymères de diallylammonium et d'acrylamide.

Dans ces compositions, ces seconds polymères peuvent être présents à une concentration comprise entre 0,01% et 10%, de préférence entre 0,03% et 5%, et encore plus préférentiellement entre 0,05% et 2%, et encore plus préférentiellement entre 0,07% et 1% du poids total de la composition.

Dans tout ce qui précède, on entend désigner par silicone non volatile ou polysiloxane, en conformité avec l'acceptation générale, tout polymère ou oligomère organosilicié à structure linéaire, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C1-C10 et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements amides, les radicaux acyloxy ou acyloxyalkyls, des groupements anioniques tels que carboxylates, thioglycolates, sulfosuccinates, thiosulfates, phosphates et sulfates, cette liste n'étant bien entendu nullement limitative (silicones dites "organomodifiées").

Par silicone non volatile, on désigne les silicones dont le nombre d'atomes de silicium est supérieure à 7.

Selon l'invention, on désigne par silicone non aminée toute silicone ne comportant pas de fonction une amine primaire, secondaire, tertiaire ou un groupement ammonium quaternaire.

Les silicones utilisables conformément à l'invention sont en particulier des polyorganosiloxanes pouvant se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press.

On utilise des silicones de préférence des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10-6 à 2,5 m2/s à 25°C et de préférence 1.10-5 à 1 m2/s.

Parmi ces polyalkylsiloxanes, on peut citer à titre non limitatif les produits commerciaux suivants :
- les huiles SILBIONE® des séries 47 et 70 047 ou les huiles MIRASIL DM commercialisées par RHODIA CHIMIE telles que par exemple l'huile 70 047 V 500 000, ou l'huile MIRASIL DM 300 000 ;
- les huiles de la série MIRASIL® DM commercialisées par la société RHODIA CHIMIE ;
- les huiles de la série 200 de la société DOW CORNING ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE .

Dans cette classe de polyalkylsiloxanes, on peut également citer les produits commercialisés sous les dénominations "ABIL® WAX 9800 et 9801" par la société GOLDSCHMIDT qui sont des polyalkyl (C1-C20) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
. les huiles SILBIONE® de la série 70 641 de RHODIA CHIMIE;
. les huiles des séries RHODORSIL 70 633 et 763 de RHODIA CHIMIE ;
. l'huile DOW CORNING 556® COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des poids moléculaires moyens en poids élevés compris entre 200 000 et 3 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylméthylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
. les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :

R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".

Les silicones organomodifiées, utilisées de préférence dans l'invention, ne comportent pas de groupements polyalkylèneoxy.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur ladite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 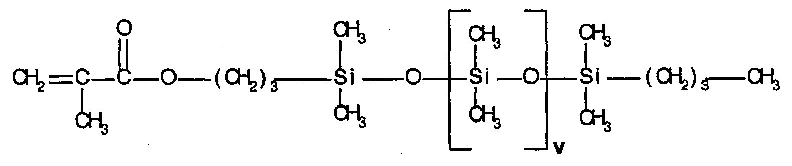
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, toutes les silicones peuvent également être utilisées sous forme d'émulsions.

Les polyorganosiloxanes particulièrement préférés conformément à l'invention sont les silicones non volatiles choisies dans la famille des polyalkylsiloxanes à groupements terminaux triméthylsilyle telles que les huiles polydiméthylsiloxane ayant une viscosité comprise entre 0,2 et 2,5 m²/s à 25° C des séries SILBIONE 70047 et 47 et plus particulièrement l'huile 70 047 V 500 000 commercialisées par la société RHODIA CHIMIE, les polyalkylsiloxanes à groupements terminaux diméthylsilanol ou les polyalkylarylsiloxanes tels que l'huile SILBIONE 70641 V 200 commercialisée par la société RHODIA CHIMIE.

Les compositions cosmétiques conformes à l'invention renferment les silicones non aminées définies ci-dessus à des teneurs pondérales qui peuvent être comprises entre 0,05 % et 10 %, de préférence entre 0,1 % et 5 % et encore plus préférentiellement entre 0,2 % et 3%, par rapport au poids total de la composition.

Le milieu approprié pour cette composition cosmétique est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants peuvent être présents dans des proportions de préférence comprises entre 1 et 40 % environ du poids total de la composition, et encore plus préférentiellement entre 5 et 30 % environ.

La composition cosmétique conforme à l'invention peut également renfermer au moins un adjuvant utilisé classiquement dans les compositions cosmétiques, choisi parmi les agents tensio-actifs cationiques, non-ioniques, ou leurs mélanges, des polymères cationiques autres que ceux de l'invention, des polymères anioniques, non-ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement autres que ceux de l'invention, des agents filmogènes, des céramides, des agents conservateurs, des agents opacifiants ou nacrants.

Ces adjuvants ci-dessus sont en général présents en quantité comprise pour chacun d'eux entre 0,01 et 20 % du poids de la composition.

Bien entendu, l'homme de l'art veillera à choisir ce ou ces éventuels composés complémentaires de manière telle que les propriétés avantageuses attachées intrinsèquement à la composition cosmétique conforme à l'invention ne soient pas, ou substantiellement pas, altérées par la ou les adjonctions envisagées.

Le pH de la composition cosmétique conforme à l'invention est généralement compris entre 2 et 12 environ, et de préférence entre 3 et 11 environ, et encore plus préférentiellement de 5 à 8.

L'invention a également pour objet un procédé de traitement cosmétique, consistant en l'application sur les fibres kératinique, d'une composition cosmétique conforme à l'invention, puis après un éventuel temps de pause de 15 secondes à 15 minutes en un rinçage desdites fibres.

Les exemples qui suivent illustrent l'invention sans en limiter la portée.

### Exemple 1

La demanderesse a préparé une composition selon l'invention et une composition de l'art antérieur. Leur formulation est définie dans le tableau ci dessous :

| Composition | invention | comparatif |
|---|---|---|
| Lauryléther sulfate de sodium à 2,2 moles d'oxyde d'éthylène | 15g M.A. | 15g M.A. |
| Cocoamidopropyl bétaïne | 2,4g M.A. | 2,4g M.A. |
| Copolymère chlorure de diméthyl diallyl | 1,5g (0,13g | 1,5g (0,13g |
| ammonium/acrylamide (50/50) en solution aqueuse à 8,7% de M.A. vendu sous la dénomination Merquat S par la société NALCO | M.A.) | M.A.) |
| Gomme de guar modifiée par du chlorure de 2,3-époxypropyltriméthylammonium vendue sous la dénomination Jaguar C13 S par la société RHODIA | 0,1 g | 0,1 g |
| Polydiméthylsiloxane vendu sous la dénomination DC 200 fluid 350 cS par la société DOW CORNING (silicone non volatile) | 2,7g | - |
| Mélange cyclopentadiméthylsiloxane /cyclohexadiméthylsiloxane vendu sous la dénomination DC345 fluid par la société Dow CORNING (silicone volatile) | - | 2,7g |
| Distéaryléther | 1,5g | 1,5g |
| Alcool béhénylique | 1,5g | 1,5g |
| Acide polyacrylique réticulé | 0,2g | 0,2g |
| Monoisopropanolamide d'acides de coprah | 1,0g | 1,0g |
| Conservateurs, parfum | q.s. | q.s. |
| Agent de pH q.s.p. | pH 7 | pH 7 |
| Eau déminéralisée q.s.p. | 100g | 100g |

Des essais comparatifs entre ces deux compositions ont été effectués sur dix modèles aux cheveux sensibilisés. Ils ont montré que la composition selon l'invention permet d'obtenir :
- une mousse plus abondante dans 70% des cas,
- un toucher moins rêche des cheveux mouillés dans 80% des cas.

De plus, la formule conforme à l'invention est stable après 2 mois de conservation à 45°C, contrairement au comparatif.

### Exemple 2

La demanderesse a préparé la composition suivante selon l'invention :

| | |
|---|---|
| Lauryléther sulfate de sodium à 2,2 moles d'oxyde d'éthylène | 15g M.A. |
| Cocoamidopropyl bétaïne | 2,4g M.A. |
| Copolymère chlorure de diméthyl diallyl ammonium/acrylamide (50/50) en solution aqueuse à 8,7% vendu sous la dénomination Merquat S par la société NALCO | 1,5g |
| Gomme de guar modifiée par du chlorure de 2,3-époxypropyltriméthylammonium vendue sous la dénomination Jaguar C13 S par la société RHODIA | 0,1g |
| Polydiméthylsiloxane vendu sous la dénomination DC 200 FLUID 300 000 par la société DOW CORNING | 2,7g |
| Distéaryléther | 1,5g |
| Alcool béhénylique | 1,5g |
| Acide polyacrylique réticulé | 0,2g |
| Monoisopropanolamide d'acides de coprah | 1,0g |
| Conservateurs, parfum | q.s. |
| Agent de pH q.s.p. | pH 7 |
| Eau déminéralisée q.s.p. | 100g |

Cette composition apporte aux cheveux bouclés du lissage et conduit à une belle définition de la boucle. Les boucles sont toniques et lisses.

## Revendications

1. Composition cosmétique pour le traitement des fibres kératiniques, telles que les cheveux, comprenant :
- au moins un tensioactif anionique ;
- au moins un tensioactif amphotère ou zwitterionique;
- au moins un premier polymère cationique choisi parmi les polysaccharides cationiques ;
- au moins un second polymère cationique choisi parmi les homo ou copolymères de dialkyldiallyl ammonium ; et
- au moins une silicone non volatile non aminée,
la quantité totale de tensioactifs étant comprise dans la composition entre 4,5 et 20 % du poids total de la composition.

2. Composition selon la revendication 1, **caractérisée en ce que** le tensioactif anionique est choisi parmi les sels de métaux alcalins, les sels d'ammonium, les sels d'amines, les sels d'aminoalcools, les sels de magnésium, les sels des composés suivants : les alkylsulfates, les alkyléthersulfates, les alkylamidoéthersulfates, les alkylarylpolyéthersulfates, les sulfates de monoglycérides ; les alkylsulfonates, les alkylphosphates, les alkylamidesulfonates, les alkylarylsulfonates, les α-oléfine-sulfonates, les paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates, les esters d'alkyl(C₆-C₂₄)polyglycosides carboxyliques, les acyliséthionates, les N-acyltaurates, les sels d'acides gras, les acyl-lactylates, les acides d'alkyl D-galactoside uroniques, les acides alkyl(C₆-C₂₄)éther carboxyliques polyoxyalkylénés, les acides alkyl(C₆-C₂₄)aryléther carboxyliques polyoxyalkylénés, les acides alkyl(C₆-C₂₄)amidoéther carboxyliques polyoxyalkylénés et leurs sels.

3. Composition selon la revendication 1 ou 2, **caractérisée en ce que** le tensioactif amphotère ou zwitterionique est choisi parmi des amines ou les ammoniums quaternaires portant une chaîne aliphatique linéaire ou ramifiée comportant 8 à 18 atomes de carbone et contenant au moins un groupe anionique hydrosolubilisant.

4. Composition selon la revendication 3, **caractérisée en ce que** le tensioactif amphotère ou zwitterionique est choisi parmi les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes, les amines de formule suivante :
R₁-CONHCH₂CH₂-N⁺(R₂)(R₃)(CH₂COO⁻)
dans laquelle :
R₁ désigne un radical alkyle linéaire ou ramifié en C₅-C₂₀ provenant d'un acide R₁-COOH présent dans l'huile de coprah hydrolysée, un radical heptyle, nonyle ou undécyle, R₂ désigne un groupement β-hydroxyéthyle et R₃ un groupement carboxyméthyle ; et
R₄-CONH CH₂CH₂-N(B)(C)
dans laquelle :
B représente -CH₂CH₂OX, C représente -(CH₂)_{z}-Y, avec z = 1 ou 2,
X désigne le groupement - CH₂CH₂-COOH ou un atome d'hydrogène,
Y désigne -COOH ou le radical -CH₂-CHOH-SO₃H,
R₄ désigne un radical alkyle linéaire ou ramifié, saturé ou non en C₅-C₂₀ provenant d'un acide R₄-COOH présent par exemple dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

5. Composition selon la revendication 4, **caractérisée en ce que** les tensioactifs amphotères ou zwitterioniques sont choisis parmi les alkylbétaïnes, les amidoalkylbétaïnes et les alkylamphodiacétates.

6. Composition selon l'une quelconque des revendications 1 à 5, **caractérisée en ce que** le premier polymère cationique est choisi parmi les celluloses cationiques et les gommes de guar cationiques.

7. Composition selon la revendication 6, **caractérisée en ce que** le premier polymère cationique est choisi parmi les dérivés d'éthers de cellulose comportant des groupements ammonium quaternaire, des dérivés de cellulose greffés avec un monomère hydrosoluble d'ammonium quaternaire, des gommes de guar contenant des groupements cationiques trialkylammonium.

8. Composition selon la revendication 7, **caractérisée en ce que** le premier polymère cationique est choisi parmi des gommes de guar modifiées par un sel de 2,3-époxypropyl triméthylammonium, de glycidyltriméthylammonium, ou de 3-chloro-2-hydroxypropyltriméthyl ammonium.

9. Composition selon l'une quelconque des revendications 1 à 8, **caractérisée en ce que** le second polymère est choisi parmi les homopolymères ou copolymères de dialkyldiallylammonium comportant comme constituant principal de la chaîne des motifs répondant aux formules suivantes (III) ou (IV) : 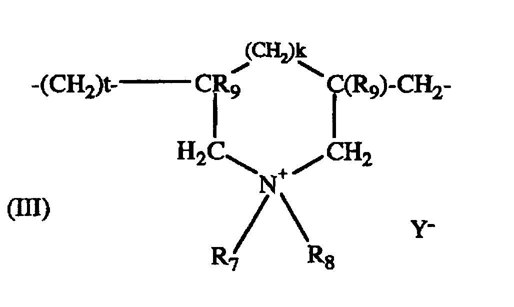 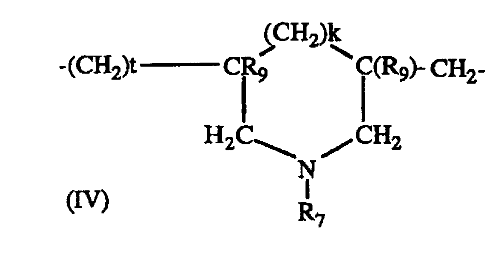 dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₇ peuvent désigner conjointement avec l'atome d'azote auquel ils sont rattachés, des groupement hétérocycliques, tels que pipéridinyle ou morpholinyle ; R₇ et R₇ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate..

10. Composition selon la revendication 9, **caractérisée en ce que** le second polymère est choisi parmi les homopolymères ou copolymères de diallyldiméthylammonium.

11. Composition selon l'une quelconque des revendications 1 à 10, **caractérisée en ce que** la silicone non volatile non aminée est choisie dans la famille des polyalkylsiloxanes à groupements terminaux triméthylsilyle.

12. Composition selon l'une quelconque des revendications 1 à 11, **caractérisée en ce que** le ou les tensioactifs anioniques sont présents dans la composition dans des concentrations allant de 0,01 à 19% du poids total de la composition.

13. Composition selon l'une quelconque des revendications 1 à 12, **caractérisée en ce que** le ou les tensioactifs amphotères ou zwitterioniques sont présents dans la composition dans des concentrations allant de 1 à 10% du poids total de la composition.

14. Composition selon l'une quelconque des revendications 1 à 13, **caractérisée en ce que** le rapport pondéral entre le(s) tensioactif(s) anionique(s) et le(s) tensioactif(s) amphotère(s) est supérieur à 1,5.

15. Composition selon l'une quelconque des revendications 1 à 14, **caractérisée en ce que** le premier polymère cationique est présent à une concentration comprise entre 0,01% et 10%, du poids total de la composition.

16. Composition selon l'une quelconque des revendications 1 à 15, **caractérisée en ce que** le second polymère cationique est présent à une concentration comprise entre 0,01 % et 10% du poids total de la composition.

17. Composition selon l'une quelconque des revendications 1 à 16, **caractérisée en ce que** la silicone non volatile non aminée est présente dans des proportions comprises entre 0,05% à 10% du poids total de la composition.

18. Composition cosmétique selon l'une quelconque des revendications 1 à 17, **caractérisée en ce qu'**elle a un pH compris entre 2 et 12.

19. Procédé de traitement cosmétique des fibres kératiniques, comprenant :
- l'application sur lesdites fibres d'une composition cosmétique telle que définie aux revendications 1 à 18,
- un temps de pause compris entre 15 secondes et 15 minutes ;
- puis un rinçage desdites fibres.

20. Utilisation de la composition cosmétique telle que définie dans les revendications 1 à 18, pour améliorer la qualité des boucles, en particulier leur tonicité.

## Patentansprüche

1. Kosmetische Zusammensetzung für die Behandlung von Keratinfasern, wie dem Haar, die Folgendes umfasst:
- mindestens ein anionisches Tensid;
- mindestens ein amphoteres oder zwitterionisches Tensid;
- mindestens ein erstes kationisches Polymer, ausgewählt aus den kationischen Polysacchariden;
- mindestens ein zweites kationisches Polymer, ausgewählt aus den Dialkyldiallylammoniumhomo- oder -copolymeren; und
- mindestens ein nichtflüchtiges nichtaminhaltiges Silikon,
wobei die Gesamttensidmenge in der Zusammensetzung zwischen 4,5 und 20% des Gesamtgewichts der Zusammensetzung ausmacht.

2. Zusammensetzung nach Anspruch 1, **dadurch gekennzeichnet, dass** das anionische Tensid aus der Reihe Alkalimetallsalze, Ammoniumsalze, Aminsalze, Aminoalkoholsalze, Magnesiumsalze, Salze der folgenden Verbindungen: Alkylsulfate, Alkylethersulfate, Alkylamidoethersulfate, Alkylarylpolyethersulfate, Monoglyceridsulfate; Alkylsulfonate, Alkylphosphate, Alkylamidsulfonate, Alkylarylsulfonate, α-Olefinsulfonate, Paraffinsulfonate, Alkyl-(C₆-C₂₄)-sulfosuccinate, Alkyl-(C₆-C₂₄) -ethersulfosuccinate, Alkyl- (C₆-C₂₄) - amidsulfosuccinate, Alkyl-(C₆-C₂₄)-sulfoacetate, Acyl-(C₆-C₂₄)-sarcosinate und Acyl-(C₆-C₂₄)-glutamate, Alkyl-(C₆-C₂₄)-polyglykosidcarbonsäureester, Acylisethionate, N-Acyltaurate, Fettsäuresalze, Acyllactylate, Alkyl-D-galactosiduronsäuren, polyoxyalkylenierte Alkyl-(C₆-C₂₄)-ethercarbonsäuren, polyoxyalkylenierte Alkyl-(C₆-C₂₄)-arylethercarbonsäuren, polyoxyalkylenierte Alkyl-(C₆-C₂₄)-Amidoethercarbonsäuren und ihren Salzen ausgewählt ist.

3. Zusammensetzung nach Anspruch 1 oder 2, **dadurch gekennzeichnet, dass** das amphotere oder zwitterionische Tensid aus der Reihe Amine oder quartäre Ammonium mit einer geradkettigen oder verzweigten aliphatischen Kette, die 8 bis 18 Kohlenstoffatome trägt und mindestens eine anionische wasserlöslich machende Gruppe enthält, ausgewählt ist.

4. Zusammensetzung nach Anspruch 3, **dadurch gekennzeichnet, dass** das amphotere oder zwitterionische Tensid aus der Reihe Alkyl-(C₈-C₂₀)-betaine, Sulfobetaine, Alkyl-(C₈-C₂₀)-amidoalkyl-(C₁-C₆)-betaine oder Alkyl-(C₈-C₂₀)-amidoalkyl-(C₁-C₆)-sulfobetaine, Amine der folgenden Formel:
R₁-CONHCH₂CH₂-N⁺(R₂) (R₃) (CH₂COO⁻),
in der:
R₁ einen geradkettigen oder verzweigten C₅-C₂₀-Alkylrest, der von einer Säure R₁-COOH, die in hydrolysiertem Kopraöl vorliegt, stammt, einen Heptylrest, Nonylrest oder Undecylrest bedeutet, R₂ eine β-Hydroxyethylgruppe bedeutet und R₃ eine Carboxymethylgruppe bedeutet; und
R₄-CONHCH₂CH₂-N (B) (C),
in der:
B -CH₂CH₂OX bedeutet, C-(CH₂)_{z}-Y bedeutet, wobei z = 1 oder 2,
X die Gruppe -CH₂CH₂-COOH oder ein Wasserstoffatom bedeutet,
Y -COOH oder den Rest -CH₂-CHOH-SO₃H bedeutet,
R₄ einen geradkettigen oder verzweigten, gesättigten oder ungesättigten C₅-C₂₀-Alkylrest, der von einer Säure R₄-COOH, die zum Beispiel in hydrolysiertem Kopraöl oder Leinöl vorkommt, stammt, einen Alkylrest, insbesondere C₇-, C₉-, C₁₁- oder C₁₃-Alkylrest, einen C₁₇-Alkylrest und seine Isoform, einen ungesättigten C₁₇-Rest bedeutet, ausgewählt ist.

5. Zusammensetzung nach Anspruch 4, **dadurch gekennzeichnet, dass** die amphoteren oder zwitterionischen Tenside aus der Reihe Alkylbetaine, Amidoalkylbetaine und Alkylamphodiacetate ausgewählt sind.

6. Zusammensetzung nach einem der Ansprüche 1 bis 5, **dadurch gekennzeichnet, dass** das erste kationische Polymer aus der Reihe kationische Cellulosen und kationische Guargummen ausgewählt ist.

7. Zusammensetzung nach Anspruch 6, **dadurch gekennzeichnet, dass** das erste kationische Polymer aus der Reihe Celluloseetherderivate mit quartären Ammoniumgruppen, Cellulosederivate, die mit einem wasserlöslichen quartären Ammonium-Monomer gepfropft sind, Guargummen, die kationische Trialkylammoniumgruppen enthalten, ausgewählt ist.

8. Zusammensetzung nach Anspruch 7, **dadurch gekennzeichnet, dass** das erste kationische Polymer aus der Reihe Guargummen, die mit einem 2,3-Epoxypropyltrimethylammoniumsalz, einem Glycidyltrimethylammoniumsalz oder einem 3-Chlor-2-Hydroxypropyltrimethyl-ammoniumsalz modifiziert sind, ausgewählt ist.

9. Zusammensetzung nach einem der Ansprüche 1 bis 8, **dadurch gekennzeichnet, dass** das zweite Polymer aus der Reihe Dialkyldiallylammoniumhomopolymere oder -copolymere, die als Hauptbestandteil der Kette Einheiten gemäß den folgenden Formeln (III) oder (IV) entsprechen, umfassen: 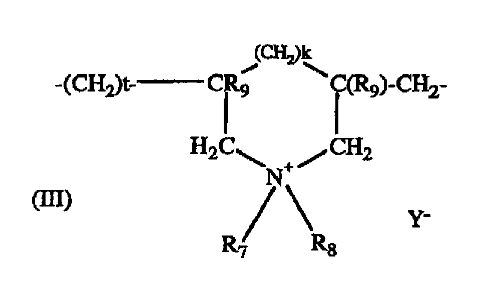 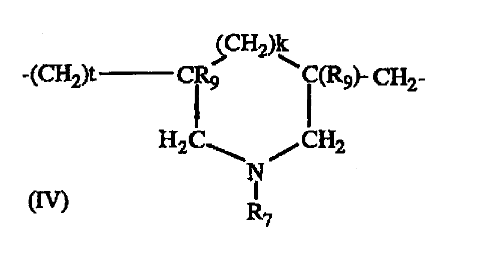 worin k und t 0 oder 1 bedeuten, die Summe von k + t gleich 1 ist; R₉ ein Wasserstoffatom oder einen Methylrest bedeutet; R₇ und R₈ unabhängig voneinander eine Alkylgruppe mit 1 bis 6 Kohlenstoffatomen, eine Hydroxyalkylgruppe, in der die Alkylgruppe vorzugweise 1 bis 5 Kohlenstoffatome aufweist, eine Amido-Niederalkyl-(C₁-C₄)-Gruppe bedeuten, oder R₇ und R₈ gemeinsam mit dem Stickstoffatom, an das sie gebunden sind, heterocyclische Gruppen wie Piperidinylgruppen oder Morpholinylgruppen bedeuten können; R₇ und R₈ unabhängig voneinander vorzugweise eine Alkylgruppe mit 1 bis 4 Kohlenstoffatomen bedeuten; Y⁻ ein Anion wie Bromid, Chlorid, Acetat, Borat, Citrat, Tartrat, Bisulfat, Bisulfit, Sulfat oder Phosphat bedeutet, ausgewählt ist.

10. Zusammensetzung nach Anspruch 9, **dadurch gekennzeichnet, dass** das zweite Polymer aus der Reihe Diallyldimethylammoniumhomopolymere oder -copolymere ausgewählt ist.

11. Zusammensetzung nach einem der Ansprüche 1 bis 10, **dadurch gekennzeichnet, dass** das nichtflüchtige nichtaminhaltige Silikon aus der Familie der Polyalkylsiloxane mit Trimethylsilylendgruppen ausgewählt ist.

12. Zusammensetzung nach einem der Ansprüche 1 bis 11, **dadurch gekennzeichnet, dass** die anionischen Tenside in der Zusammensetzung in Konzentrationen von 0,01 bis 19% des Gesamtgewichts der Zusammensetzung vorliegen.

13. Zusammensetzung nach einem der Ansprüche 1 bis 12, **dadurch gekennzeichnet, dass** das amphotere Tensid oder zwitterionische Tensid bzw. die amphoteren Tenside oder zwitterionischen Tenside in der Zusammensetzung in Konzentrationen von 0 bis 10% des Gesamtgewichts der Zusammensetzung vorliegt bzw. vorliegen.

14. Zusammensetzung nach einem der Ansprüche 1 bis 13, **dadurch gekennzeichnet, dass** das Gewichtsverhältnis zwischen dem anionischen Tensid bzw. den anionischen Tensiden und dem amphoteren Tensid bzw. den amphoteren Tensiden mehr als 1,5 beträgt.

15. Zusammensetzung nach einem der Ansprüche 1 bis 14, **dadurch gekennzeichnet, dass** das erste kationische Polymer in einer Konzentration von zwischen 0,01% und 10% des Gesamtgewichts der Zusammensetzung vorliegt.

16. Zusammensetzung nach einem der Ansprüche 1 bis 15, **dadurch gekennzeichnet, dass** das zweite kationische Polymer in einer Konzentration von zwischen 0,01% und 10% des Gesamtgewichts der Zusammensetzung vorliegt.

17. Zusammensetzung nach einem der Ansprüche 1 bis 16, **dadurch gekennzeichnet, dass** das nichtflüchtige nichtaminhaltige Silikon in Verhältnissen zwischen 0,05% bis 10% des Gesamtgewichts der Zusammensetzung vorliegt.

18. Kosmetische Zusammensetzung nach einem der Ansprüche 1 bis 17, **dadurch gekennzeichnet, dass** sie einen pH-Wert zwischen 2 und 12 aufweist.

19. Verfahren für die kosmetische Behandlung von Keratinfasern, das Folgendes umfasst:
- Applizieren einer kosmetischen Zusammensetzung wie in den Ansprüchen 1 bis 18 definiert auf diese Fasern,
- Einhalten einer Pause von zwischen 15 Sekunden und 15 Minuten;
- anschließendes Spülen dieser Fasern.

20. Verwendung der kosmetischen Zusammensetzung wie in den Ansprüchen 1 bis 18 definiert für die Verbesserung der Lockenqualität, insbesondere ihrer Sprungkraft.

## Claims

1. Cosmetic composition for treating keratin fibres, such as the hair, comprising:
- at least one anionic surfactant;
- at least one amphoteric or zwitterionic surfactant;
- at least one first cationic polymer chosen from cationic polysaccharides;
- at least one second cationic polymer chosen from dialkyldiallylammonium homopolymers or copolymers; and
- at least one non-amino non-volatile silicone, the total amount of surfactants in the composition being between 4.5% and 20% of the total weight of the composition.

2. Composition according to Claim 1, **characterized in that** the anionic surfactant is chosen from alkali metal salts, ammonium salts, amine salts, amino alcohol salts or magnesium salts, of the following compounds: alkyl sulfates, alkyl ether sulfates, alkylamido ether sulfates, alkylarylpolyether sulfates, monoglyceride sulfates; alkyl sulfonates, alkyl phosphates, alkylamide sulfonates, alkylaryl sulfonates, α-olefin sulfonates, paraffin sulfonates; (C₆-C₂₄)alkyl sulfosuccinates, (C₆-C₂₄)alkyl ether sulfosuccinates, (C₆-C₂₄) alkylamide sulfosuccinates; (C₆-C₂₄) alkyl sulfoacetates; (C₆-C₂₄)acyl sarcosinates; and (C₆-C₂₄)acyl glutamates, (C₆-C₂₄) alkylpolyglycoside carboxylic esters, acyl isethionates, N-acyl taurates, fatty acid salts, acyl lactylates, alkyl D-galactoside uronic acids, polyoxyalkylenated (C₆-C₂₄)alkyl ether carboxylic acids, polyoxyalkylenated (C₆-C₂₄)alkylaryl ether carboxylic acids and polyoxyalkylenated (C₆-C₂₄)alkylamido ether carboxylic acids, and the salts thereof.

3. Composition according to Claim 1 or 2, **characterized in that** the amphoteric or zwitterionic surfactant is chosen from amines or quaternary ammoniums bearing a linear or branched aliphatic chain containing from 8 to 18 carbon atoms and containing at least one water-solubilizing anionic group.

4. Composition according to Claim 3, **characterized in that** the amphoteric or zwitterionic surfactant is chosen from (C₈-C₂₀)alkylbetaines, sulfobetaines, (C₈-C₂₀) alkylamido(C₁-C₆) alkylbetaines or (C₈-C₂₀) alkylamido (C₁-C₆)alkylsulfobetaines, amines of formula:
R₁-CONHCH₂CH₂-N⁺(R₂) (R₃) (CH₂COO⁻)
in which:
R₁ denotes a C₅-C₂₀ linear or branched alkyl radical derived from an acid R₁-COOH present in hydrolysed coconut oil, a heptyl, nonyl or undecyl radical, R₂ denotes a beta-hydroxyethyl group and R₃ denotes a carboxymethyl group; and
R₄-CONH CH₂CH₂-N(B) (C)
in which:
B represents -CH₂CH₂OX, C represents -(CH₂)_{z}-Y, with z = 1 or 2,
X denotes the -CH₂CH₂-COOH group or a hydrogen atom,
Y denotes -COOH or the -CH₂-CHOH-SO₃H radical,
R₄ denotes a C₅-C₂₀, saturated or unsaturated, linear or branched alkyl radical derived from an acid R₄-COOH present for example in coconut oil or in hydrolysed linseed oil, an alkyl radical, in particular a C₇, C₉, C₁₁ or C₁₃ alkyl radical, a C₁₇ alkyl radical and its iso form, or an unsaturated C₁₇ radical.

5. Composition according to Claim 4, **characterized in that** the amphoteric or zwitterionic surfactants are chosen from alkylbetaines, amidoalkylbetaines and alkylamphodiacetates.

6. Composition according to any one of Claims 1 to 5, **characterized in that** the first cationic polymer is chosen from cationic celluloses and cationic guar gums.

7. Composition according to Claim 6, **characterized in that** the first cationic polymer is chosen from cellulose ether derivatives comprising quaternary ammonium groups, cellulose derivatives grafted with a water-soluble quaternary ammonium monomer, and guar gums containing trialkylammonium cationic groups.

8. Composition according to Claim 7, **characterized in that** the first cationic polymer is chosen from guar gums modified with a 2,3-epoxypropyltrimethylammonium, glycidyltrimethylammonium or 3-chloro-2-hydroxypropyltrimethylammonium salt.

9. Composition according to any one of Claims 1 to 8, **characterized in that** the second polymer is chosen from dialkyldiallylammonium homopolymers or copolymers comprising, as main constituent of the chain, units corresponding to formula (III) or (IV) below: 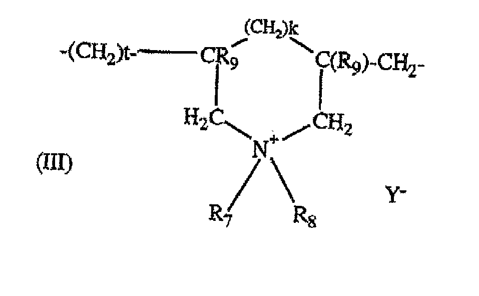 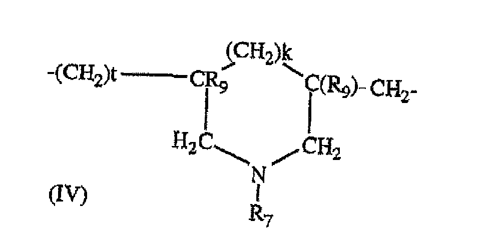 in which k and t are equal to 0 or 1, the sum k + t being equal to 1; R₉ denotes a hydrogen atom or a methyl radical; R₇ and R₈, independently of each other, denote an alkyl group containing from 1 to 6 carbon atoms, a hydroxyalkyl group in which the alkyl group preferably contains from 1 to 5 carbon atoms, a lower (C₁-C₄) amidoalkyl group, or R₇ and R₈ can denote, together with the nitrogen atom to which they are attached, heterocyclic groups such as piperidyl or morpholinyl; R₇ and R₈, independently of each other, preferably denote an alkyl group containing from 1 to 4 carbon atoms; Y⁻ is an anion such as bromide, chloride, acetate, borate, citrate, tartrate, bisulfate, bisulfite, sulfate or phosphate.

10. Composition according to Claim 9, **characterized in that** the second polymer is chosen from diallyldimethylammonium homopolymers or copolymers.

11. Composition according to any one of Claims 1 to 10, **characterized in that** the non-amino non-volatile silicone is chosen from the family of polyalkylsiloxanes containing trimethylsilyl end groups.

12. Composition according to any one of Claims 1 to 11, **characterized in that** the anionic surfactant(s) are present in the composition in concentrations ranging from 0.01% to 19% of the total weight of the composition.

13. Composition according to any one of Claims 1 to 12, **characterized in that** the amphoteric or zwitterionic surfactant(s) are present in the composition in concentrations ranging from 1% to 10% of the total weight of the composition.

14. Composition according to any one of Claims 1 to 13, **characterized in that** the weight ratio between the anionic surfactant(s) and the amphoteric surfactant(s) is greater than 1.5.

15. Composition according to any one of Claims 1 to 14, **characterized in that** the first cationic polymer is present in a concentration of between 0.01% and 10% of the total weight of the composition.

16. Composition according to any one of Claims 1 to 15, **characterized in that** the second cationic polymer is present in a concentration of between 0.01% and 10% of the total weight of the composition.

17. Composition according to any one of Claims 1 to 16, **characterized in that** the non-amino non-volatile silicone is present in proportions of between 0.05% and 10% of the total weight of the composition.

18. Cosmetic composition according to any one of Claims 1 to 17, **characterized in that** it has a pH of between 2 and 12.

19. Cosmetic process for treating keratin fibres, comprising:
- the application to the said fibres of a cosmetic composition as defined in Claims 1 to 18;
- a leave-in time of between 15 seconds and 15 minutes;
- followed by rinsing of the said fibres.

20. Use of the cosmetic composition as defined in Claims 1 to 18, to improve the quality of curls, in particular their tonicity.
